(19) Europäisches Patentamt

European Patent Office

Office européen des brevets

(11) EP 0 962 019 B1

(12) **EUROPEAN PATENT SPECIFICATION**

(45) Date of publication and mention
of the grant of the patent:
**05.12.2001 Bulletin 2001/49**

(21) Application number: **98909482.6**

(22) Date of filing: **16.02.1998**

(51) Int Cl.[7]: **G21F 9/04**, G01N 33/18,
G01T 1/178

(86) International application number:
**PCT/EP98/01118**

(87) International publication number:
**WO 98/37562 (27.08.1998 Gazette 1998/34)**

(54) **A METHOD OF PROVIDING A MEASURE OF THE TRITIUM CONTENT OF A WASTE BATCH**

METHODE ZUM MESSEN VON DEM TRITUM INHALT IN EINER ABFÄLLELADUNG

PROCEDE D'OBTENTION D'UNE MESURE DE LA TENEUR EN TRITIUM D'UN LOT DE DECHETS

(84) Designated Contracting States:
**DE FR GB**

(30) Priority: **18.02.1997 GB 9703377**

(43) Date of publication of application:
**08.12.1999 Bulletin 1999/49**

(73) Proprietor: **EUROPEAN ATOMIC ENERGY
COMMUNITY (EURATOM)
2920 Luxembourg (LU)**

(72) Inventor: **EDWARDS, Robert, Arthur, Henry
I-21027 Ispra (IT)**

(74) Representative: **Baldock, Sharon Claire et al
BOULT WADE TENNANT,
Verulam Gardens
70 Gray's Inn Road
London WC1X 8BT (GB)**

(56) References cited:
**WO-A-97/35183          CH-A- 460 188**

• **WOOD M J ET AL: "TRITIUM SAMPLING AND
MEASUREMENT" HEALTH PHYSICS, vol. 65, no.
6, December 1993, pages 610-627, XP000423640**
• **ANTONAZZI A.B. & SHMAYDA W.T.:
"Assessment of Tritium Inventory in Waste by
Outgassing Measurements", FUSION
TECHNOLOGY, , , vol. 28, no. 3, pages 1457 to
1462**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The present invention relates to a method of providing a measure of the tritium content of a batch of waste material and in particular to a batch of "soft" waste material.

Tritium is an isotope of hydrogen which undergoes radioactive decay. When waste is contaminated with tritium it is generally only defined as radioactive when the level of activity is above a certain (very low) level, which level is determined individually according to the appropriate regulatory authorities of respective countries and which vary from one country to the next. Above this level, the precautions required, and hence the expense, for the disposal of a batch of waste, increases according to the level of radioactivity of the waste. It is therefore important to define accurately the tritium activity of the waste batch in order to provide safe and economic disposal. For example, at present, a lot of waste must be disposed of as radioactive waste only because there is a theoretical possibility of it having been contaminated with tritium. This is because analytical techniques are not at present sensitive enough to detect tritium in waste at a level which defines non-radioactive status. Therefore it would be advantageous to be able to provide an assessment of the level of tritium contamination of a waste batch.

Furthermore, if any waste batch is to be incinerated it would also be desirable to know the level of tritium contamination of the waste in order to predict or estimate environmental tritium emissions.

"Soft" waste is the name given to the type of waste, typically produced by maintenance and laboratory operations, consisting mostly of disposable items of paper, plastic sheet, rubber or the like. The essential features of soft waste (in contrast to "hard" waste such as metal, glass, ceramics, or the like), which render it suitable for the application of the present invention, is that tritium is generally retained substantially in the form of absorbed or adsorbed tritiated water (HTO), and which can diffuse throughout the material in the batch.

The waste is usually stored in heat sealed polyethylene bags or the like, which may themselves be further stored in sealed drums, to reduce tritium leakage. The level of tritium contamination is usually assessed before placing the waste in the bags in the drums.

Previous methods used for estimating the tritium content in waste batches are disclosed in 1) "Assessment of tritium inventory in waste by outgassing measurements", Antoniazzi A.B., Shmyda W.T., Fusion Technology Vol.28, No.3, part 2, p.1457, and 2) "Low level tritium assessment in JET solid waste materials", Pacenti P., Atkins G., Campi F., Newbert G., Terrani S., Proc. 18th symposium on Fusion Technology, Karlsruhe, Germany 22-26 August 1994. 57.

According to reference number 1 above, the level of tritium is assessed by firstly passing humid air through a bag of waste, and then through a water bubbler, which bubbler accumulates any tritiated water outgassed. The tritiated water content in the bubbler may then be measured at various times using liquid scintillation counting. The tritium content (or tritium inventory) of the bag may then be calculated from an empirical correlation between outgassing rate and inventory. Disadvantages associated with using this technique are firstly, several liquid scintillation measurements are required to establish the slope of the cumulative-tritium-against-time curve. Secondly, the sensitivity of this method is generally reduced by the dilution of the tritium in the water bubbler. Furthermore, absorbent components of soft waste generally outgas up to three orders of magnitude slower than non-absorbent components, which can cause errors in deducing tritium inventory from outgassing rate.

According to reference number 2 above, a sample of the waste to be disposed of, for example a finger of a surgical glove or a tissue or the like, is placed in a silica tube. Any water therein is then extracted by heating the tube in a gas stream. The water vapour is subsequently collected by condensation or bubbling the off-gas through water. The tritium content of the water in the bubbler may then be measured using standard techniques, such as for example, liquid scintillation counting.

This method suffers from the disadvantages that the accuracy of the measurement of the tritium content of the waste batch is dependent on how representative the tritium content of the sample used is of the tritium in the batch as a whole. For example, 99% of any tritiated water may be maintained in a single item having a high water content. If this item is not used as the sample, the measurement obtained may not be representative of the level of tritium in the waste batch. Furthermore, the sensitivity of the method is limited by the small amount of water in the samples and the large dilution of this sample water in the bubbler.

Therefore, it is the purpose of the present invention to alleviate such difficulties and to provide a method of providing a measure of the tritium content of a waste batch which measure may be obtained from a single sample taken from the waste batch.

The present invention is based on the inventor's surprising observation, that in a container of soft waste in a polyethylene bag or the like, the ratio of any tritiated water to water in the container is constant throughout substantially all of the moisture contained in the waste batch after a period of time for equilibration has elapsed and particularly after a period of between approximately 24 to 48 hours. Therefore, a sample of moisture taken from inside this container after the tritiated water to water ratio has equalised should yield a value of this ratio which is representative of all of the waste in the container.

Therefore, according to a first aspect of the present invention there is provided a method of providing a measure of the tritium content of a waste batch, which method comprises:

a) extracting a sample of water from a sealable container having the waste batch therein using a hygroscopic sampler, which container has been sealed for a sufficient time period to enable the tritiated water (HTO) to water ratio to substantially equalise throughout the container,
b) measuring the tritium content of said sample
c) providing a measure of the total water content of the waste batch prior to or subsequent to steps a) or b), the measure of tritium in said waste, being represented by the following formula,

HTO/H$_2$O (sample) x (total water content of waste batch)

= tritium content of waste batch.

Thus, advantageously only a single sample may be required to obtain a measure of the tritium content of the waste batch. This sample provides a measure of the tritium content of the waste batch, because the HTO/H$_2$O ratio of the sample analysed is generally representative of the whole waste batch.
The method according to the present invention is more sensitive than previous methods because the water sample taken from the container is not unnecessarily diluted and the measurements taken more accurately reflect the tritiated water (HTO) to water ratio of whole waste batch.
According to a second aspect of the present invention, there is provided a method of providing a measure of the tritium content of a waste batch, which method comprises,

a) extracting a sample of water from a sealable having the waste batch therein using a hygroscopic sampler, which container has been sealed for a sufficient time period to allow hydrogen isotopes in the water content of the waste batch to substantially equalise, a known quantity of heavy water having been introduced into the container sufficiently prior to said extraction for isotopic equalisation throughout the waste to occur;

b) measuring the Deuterium/(Hydrogen+Deuterium) and the Tritium/(Hydrogen+Deuterium) ratio of the sample, wherein the measure of tritium in the waste batch is represented by the formula

$$\text{Total tritium of waste batch=}$$
$$\text{D in sample} \quad \text{x} \quad \left\{ \frac{T/(H+D) \; ratio}{D/(H+D) \; ratio} \right\}$$

The ratios of T/(H+D) and D/(H+D) are those measured in the hygroscopic sample. Since these ratios are measured after isotopic equalisation they are the same as those ratios of T/(H+D) and D/(H+D) in the batch of waste.
Furthermore, the heavy water may be added by means of injection or by previously introducing into said hygroscopic sampler said heavy water prior to introducing said sampler into the container. This may be achieved by absorbing or introducing by means of isotopic exchange a known quantity of heavy water into the hygroscopic sampler prior to inserting it into the container.
The hygroscopic sampler comprises the following characteristics,

a) it absorbs a significant percentage of moisture at room temperature in a range of relative humidity between about 10% and 90%;
b) preferably remains substantially solid under the conditions in step a);
c) allows exchange of any absorbed water with any moisture in the waste container in a convenient time period;
d) releases any water by dissolution, isotopic exchange with water or on moderate heating.

Preferably, the hygroscopic sampler comprises a water-sorbent, such as silica gel, zeolite or hygroscopic oxide (e.g. activated alumina) or even more preferably a hygroscopic salt. The hygroscopic salt is particularly advantageous because any water absorbed from the waste batch may be relatively easily extracted by dissolving the salt in water. A salt which is particularly preferable is barium chloride which advantageously has a fixed hydration state of BaCl$_2$.2H$_2$O under the conditions foreseeable inside a container of soft waste (10 to 90% relative humidity) and which is easily soluble in water.
Preferably, the hygroscopic sampler may be held within a substantially porous container, such as a paper or cloth sachet. Thus, advantageously the hygroscopic salt may be easily removable from the sealable container of step a). Preferably the hygroscopic sampler may be tethered or otherwise located in, for example a corner of the sealable container to facilitate its removal therefrom.

The tritium content of the sample of water extracted in step b) of the first embodiment of the invention may be measured using standard techniques, such as liquid scintillation counting.

The total amount of water in the container according to the first embodiment of the invention may be ascertained by either, a) assuming a typical water content based on previous measurements of the type of waste material present (which is generally sufficient for most waste batches), b) an alternative technique such as microwave absorption measurements. The container may comprise a polyethylene bag or drum used in the disposal of radioactive waste. Adopting the tracer technique as described below obviates the need for ascertaining the total amount of water in the containers.

The hygroscopic sampler material may, for example be contained in a rigid perforated holder which can be inserted into a port in the waste container to allow contact with the atmosphere around said waste. This embodiment may be preferable where the waste container is rigid, for example a drum or a can.

The method may advantageously be applied to measuring the tritium content between an outer container, such as a drum or can and one or more inner containers, such as in sealed plastic bags or a smaller drum. In this case the tritium measured indicates the amount of tritium which has leaked through the inner container(s) after closure of the outer container. The total water content needed for this measurement can be estimated on the basis of the inter-volume and the humidity of the air at the time of closure.

The invention will now be described with reference to the specific examples given by way of example only.

Example 1

1. A sachet containing, 0.15g $BaCl_2.2H_2O$ is sealed in the container of soft waste and left for $\geq$ 48 hours, to allow the hydrogen isotopes to equilibrate inside the container.

2. The sachet is subsequently extracted from the container after 48 hours and the container resealed.

3. The contents of the sachet are dissolved in 0.5ml water.

4. The tritium activity of the solution is obtained, using liquid scintillation counting.

5. The amount of water associated with the sample is calculated from the formula of the hydrated salt. The ratio of $HTO/H_2O$ in the sample was then calculated. This ratio is the same as $HTO/H_2O$ ratio in the rest of the soft waste batch.

6. The water content of the soft waste batch is estimated by experience of the typical water content form previous determinations (e.g. paper waste typically contains 20% water).

7. Total HTO inventory of soft waste batch

$$= (HTO/H_2O \text{ ratio}) \text{ x (total water content in the soft}$$

$$\text{waste batch).}$$

(The experimental validation for this test procedure is described in appendix 1).

Variations on the test procedure

Referring to steps 1 to 7 above;

Between steps 2 and 3, or 3 and 4, the sample may be kept in a sealed container (e.g. polythene bag) for later analysis at the convenience of the operator.

To minimize isotopic dilution of the sample in step 3, and to reduce the amount of solution added to the scintillator cocktail (causing quenching) the hydrated salt may be left for a few hours in a quantity of water insufficient to dissolve all of it, yet allowing the tritiated water to be extracted by exchange.

It is desirable to compensate for the quenching effect of the salt on the liquid scintillation counting, using a control of known tritium content. The quenching effect of barium chloride is not so bad as to seriously reduce sensitivity.

Alternatively, quenching by the salt can be avoided by extracting the water from the hydrated salt sachet by heating the salt and condensing the evolved water into the scintillator vial. It is sufficient to heat the barium chloride to 113°C.

For repeatability, the salt solution can be divided between various scintillation vials; more than one sampler may also be used.

The water content of the soft waste batch may also be calculated using the tracer method as described in example 2.

Example 2

By using deuterium as a tracer, the need to estimate the total water content of the waste is eliminated.

1. Equilibrate a sachet of (preferably anhydrous) barium chloride with pure heavy water ($D_2O$) vapour.

2. Use this sachet to carry out steps 1 and 2 as described in Example 1.

3. Measure the D/H ratio in the water of crystallisation. This may be done by nuclear magnetic resonance (NMR) measurements, or by extracting the water by heating, and then analysing the D/H ratio using mass spectrometry, using one of the methods described in the introduction of: O.Bréas et.al. "Deuterium/hydrogen measurements in wines and fruit juices by platinum catalysed equilibration method", Rapid communications in mass spectrometry, vol. 10, p.246-249 (1996).

$$(\text{total T inventory in the batch})=$$

$$(\text{D introduced before sealing}) \times (\text{T/[H+D]ratio})/$$

$$(\text{D/[H+D]ratio})$$

Variations on Example 2

Instead of equilibrating the barium chloride with heavy water, one may simply inject a known quantity of heavy water into the waste container when the sampler is inserted (or at any time before removal of the sampler which gives enough time for isotopic equalisation throughout the waste).

If heavy water could be already present in the batch, one may amend the procedure as follows:

- estimate first the starting D/(H+D) ratio, using a standard hygroscopic sampler and mass spectrometry, then carry out the tracer procedure as above. In the calculation, one uses the change in D/(H+D) ratio instead of the final D/ (H+D) ratio.

The same modification may be adopted if the ratio ($D_2O$ tracer added)/(total water in the waste) is so low that the D/(H+D) ratio approaches the natural isotopic deuterium abundance in atmospheric water (about 0.0156%). However, it is usually easier to simply increase the quantity of heavy water tracer added to overcome this problem.

Experimental validation of the test procedure in Example 1

1. A polythene bag containing 1kg of uncontaminated laboratory soft waste, comprising tissues, disposable gloves and polythene sheeting, was prepared using a heat-sealing machine, which melts together the upper and lower plastic sheets along a narrow strip. The heat-sealer was also used to melt together the plastic in a series of parallel strips terminating at one sealed edge of the bag, so as to form 6 tubes or "fingers", suitable for retaining the sampler sachets, communicating with the main space inside the bag.

2. The water content of the waste was estimated by weighing an identical collection of waste before and after drying at 120°C. The total water content of the waste in the bag was estimated to be 11g.

3. Just before the bag was sealed, 50 microlitres of tritiated water was injected into the waste using a micropipette (it was immediately soaked up). The bag was then kept sealed for a week to allow isotopic equilibration. The amount of tritium in an identical sample of tritiated water was measured using liquid scintillation counting. The total tritium content of the waste bag was thus known to be $5.2\pm5$ MBq.

4. 6 paper sachets containing 0.15g each of $BaCl_2.2H_2O$ were prepared. The waste bag was cut along one edge to reveal the "fingers". One sachet was inserted into each "finger". Care was taken to avoid squeezing air out of the bag during this process. The "fingers" were then re-sealed.

5. At 10 mins, 1h, 4h, 9h, 32h and 74 hours after re-sealing, a "finger" containing a sachet was sealed off from the rest of the waste bag using the heat sealer, cut open and the sachet extracted. The contents of the sachet were immediately dissolved in 0.5ml water, in a scintillation vial. 5ml of scintillation cocktail were added and the vial was counted in a scintillation counter.

6. The effect of barium chloride on the efficiency of scintillation counting was determined by counting a vial containing 5ml scintillation cocktail + 0.15g $BaCl_2.2H_2O$ + 0.5ml of tritiated water of known activity. The measured counting efficiency (uncorrected counts-per-second/Bq of tritium) was 0.135, compared to 0.58 without the barium chloride.

7. The tritium content of the sachets increased sharply with time-before-extraction at first, but the increase levelled off, so that the sachet extracted after 4h already had half as much tritium as in the final sachet extracted after 74h, whereas the tritium in the sachet extracted at 32h was only two percent less than in the final sachet. The conclusion is that after about two days the sachets have reached within 2% the equilibrium isotopic dilution of the water in the waste.

8. Taking into account the measured efficiency of the scintillation counting, the tritium content of the 72h sachet was calculated to be 1015Bq.
0.15g of $BaCl_2.2H_2O$ contains 0.022g water: this sampled mass of water compares to a total water content (see step 2) in the waste of 11g (the contribution of water in the sachets is negligible). Therefore the total tritium content in the waste is estimated to be:

$$1015 \times 11 \div 0.022 = 5.1 \times 10^5 \text{ Bq}$$

..... which is within the error range of the amount of tritiated water used to artificially contaminate the waste in step 3.

The ultimate sensitivity of the invention depends on the noise background of the scintillation counter. For a counting time of 20 minutes, the 3-sigma statistical fluctuation in background of our counter was 0.04 counts-per-second. In this application of the invention the counting efficiency was 0.135 which gives a limit of tritium detection of 0.3Bq in the sampler, corresponding to a limit of tritiated water detection in this waste bag (11g water in 1kg waste) of $(0.3 \times 11/0.022)/1000 = 0.148\Box$Bq/g waste. This is below the limit for declassification of tritiated waste in the United Kingdom (0.5 Bq/g).

## Claims

**1.** A method of providing a measure of the tritium content of a waste batch, which method comprises;

a) extracting a sample of water from a sealable container having said waste therein using a hygroscopic sampler, said container being sealed for a time period sufficient to allow the tritiated water to water ratio to substantially equalise throughout the container,
b) measuring the tritium content of said sample,
c) providing a measure of the total water content of said waste batch prior to or subsequent to steps a) or b),

the measure of tritium of said waste being represented by the following formula:

$$HTO/H_2O \text{ (sample) x total water content of waste batch}$$

= tritium content of waste batch.

2. A method of providing a measure of the tritium content of a waste batch, which method comprises,

a) extracting a sample of water from a sealable container having said waste therein using a hygroscopic sampler, which container has been sealed for a sufficient time period to allow substantial equalisation of the hydrogen isotope distribution in the water component of the container contents, a known quantity of heavy water having been introduced into the container sufficiently prior to said extraction for isotopic equalisation throughout the waste to occur,

b) measuring the D/(H+D) and T/(H+D) ratio of the sample, wherein the measure of the tritium in the batch is represented by the formula:

$$\text{Total tritium in waste batch} = (\text{D in sample}) \times \left\{ \frac{T \ / \ [H \ + \ D] \ ratio}{D \ / \ [H \ + \ D] \ ratio} \right\}$$

3. A method according to claim 2 or 3 wherein said heavy water is introduced into said container by means of said hygroscopic sampler.

4. A method according to any of claims 1 to 3, wherein said hygroscopic sampler comprises any of the following characteristics:

a) it absorbs a significant percentage of water at room temperature, in a range of relative humidity between approximately 10 and 90%,
b) remains substantially solid under the conditions defined in a),
c) allows exchange of any absorbed water with any moisture in the remainder of the waste batch,
d) releases the water by dissolution or moderate heating, or by isotopic exchange within a convenient time period.

5. A method according to any of claims 1 to 4, wherein said hygroscopic sampler is provided in a substantially porous or perforated container.

6. A method according to claim 5, wherein said porous container is a paper or cloth sachet.

7. A method according to any preceding claim wherein said hygroscopic sampler is provided in a perforated holder suitable for fitting through an entry port in said waste container.

8. A method according to any preceding claim wherein said hygroscopic sampler is introducable into or removable from said container without breaking the seal of said waste container.

9. A method according to any preceding claim wherein said hygroscopic sampler comprises any of a hygroscopic salt, hygroscopic oxide, silica gel or zeolite.

10. A method according to any preceding claim wherein said hygroscopic salt is barium chloride.

11. A method according to any preceding claim wherein said sealable waste container comprises a bag or drum.

**Patentansprüche**

1. Verfahren zum Bereitstellen eines Maßes des Tritiumgehalts einer Abfallcharge, wobei das Verfahren die folgenden Schritte aufweist:

a) Extrahieren einer Wasserprobe aus einem dicht verschließbaren Behälter, der den Abfall darin enthält, unter Verwendung eines hygroskopischen Probenehmers, wobei der Behälter für einen Zeitraum dicht verschlossen ist, der ausreicht, um zuzulassen, daß sich das Verhältnis von mit Tritium behandeltem Wasser zu Wasser im gesamten Behälter im wesentlichen ausgleicht;
b) Messen des Tritiumgehalts der Probe,
c) Bereitstellen eines Maßes des Gesamtwassergehalts der Abfallcharge vor oder nach den Schritten a) oder b), wobei das Maß von Tritium des Abfalls durch die folgende Formel dargestellt ist:

HTO/H$_2$O (Probe) x Gesamtwassergehalt der Abfallcharge

= Tritiumgehalt der Abfallcharge.

2. Verfahren zum Bereitstellen eines Maßes des Tritiumgehalts einer Abfallcharge, wobei das Verfahren die folgenden Schritte aufweist:

a) Extrahieren einer Wasserprobe aus einem dicht verschließbaren Behälter, der den Abfall darin enthält, unter Verwendung eines hygroskopischen Probenehmers, wobei der Behälter für einen Zeitraum dicht verschlossen worden ist, der ausreicht, um zuzulassen, daß sich die Wasserstoffisotopenverteilung in der Wasserkomponente des Behälterinhalts im wesentlichen ausgleicht, wobei eine bekannte Menge an schwerem Wasser in den Behälter eingebracht worden ist, und zwar ausreichend lang vor dem Eintreten der Extraktion zum Zweck des isotopen Ausgleichs im gesamten Abfall,
b) Messen des Verhältnisses D/(H+D) und T/(H+D) der Probe, wobei das Maß des Tritiums in der Charge durch die folgende Formel dargestellt ist:

Gesamttritium in der Abfallcharge = (D in der Probe) x $\left\{\dfrac{T/[H+D] \text{ Verhältnis}}{D/[H+D] \text{ Verhältnis}}\right\}$

3. Verfahren nach Anspruch 1 oder 2, wobei das schwere Wasser durch den hygroskopischen Probenehmer in den Behälter eingebracht wird.

4. Verfahren nach einem der Ansprüche 1 bis 3, wobei der hygroskopische Probenehmer eine oder jede der folgenden Eigenschaften aufweist:

a) er absorbiert einen signifikanten Prozentsatz an Wasser bei Raumtemperatur in einem Bereich relativer Feuchte zwischen ungefähr 10 und 90%,
b) bleibt unter den in a) definierten Bedingungen im wesentlichen fest,
c) erlaubt den Austausch von etwaigem absorbiertem Wasser mit etwaiger Feuchtigkeit in dem Rest der Abfallcharge,
d) setzt das Wasser durch Auflösen oder mäßiges Erwärmen oder durch isotopen Austausch innerhalb eines angemessenen Zeitraums frei.

5. Verfahren nach einem der Ansprüche 1 bis 4, wobei der hygroskopische Probenehmer in einem im wesentlichen porösen oder perforierten Behälter vorgesehen ist.

6. Verfahren nach Anspruch 5, wobei der poröse Behälter ein Papier- oder Tuchbeutel ist.

7. Verfahren nach einem der vorhergehenden Ansprüche, wobei der hygroskopische Probenehmer in einem perforierten Halter vorgesehen ist, der zum Einsetzen durch eine Eintrittsöffnung in dem Abfallbehälter geeignet ist.

8. Verfahren nach einem der vorhergehenden Ansprüche, wobei der hygroskopische Probenehmer ohne ein Aufbrechen des dichten Verschlusses des Abfallbehälters in den Behälter einführbar oder aus diesem entnehmbar ist.

9. Verfahren nach einem der vorhergehenden Ansprüche, wobei der hygroskopische Probenehmer eines von einem hyygroskopischen Salz, einem hygroskopischen Oxid, einem Kieselgel oder einem Zeolith aufweist.

**10.** Verfahren nach einem der vorhergehenden Ansprüche, wobei das hygroskopische Salz Bariumchlorid ist.

**11.** Verfahren nach einem der vorhergehenden Ansprüche, wobei der dicht verschließbare Abfallbehälter einen Sack oder eine Trommel aufweist.

**Revendications**

**1.** Procédé d'obtention d'une mesure de la teneur en tritium dans un lot de déchets, lequel procédé comprend les étapes consistant à :

a) extraire un échantillon d'eau depuis un conteneur scellable contenant lesdits déchets, en utilisant un échantillonneur hygroscopique, ledit conteneur étant scellé pendant une période suffisante pour permettre au rapport eau tritiée/eau de sensiblement s'égaliser au sein du conteneur ;
b) mesurer la teneur en tritium dudit l'échantillon, et
c) obtenir une mesure de la teneur totale en eau dudit lot de déchets, avant ou après les étapes a) ou b),

la mesure du tritium desdits déchets étant représentée par la formule suivante :

$$HTO/H_2O \text{ (échantillon)} \times \text{teneur totale en eau du lot de}$$

$$\text{déchets} = \text{teneur en tritium dans le lot de déchets.}$$

**2.** Procédé d'obtention d'une mesure de la teneur en tritium dans un lot de déchets, lequel procédé comprend les étapes consistant à :

a) extraire un échantillon d'eau depuis un conteneur scellable contenant lesdits déchets, en utilisant un échantillonneur hygroscopique, ledit conteneur ayant été scellé pendant une période suffisante pour permettre une sensible égalisation de la distribution isotopique de l'hydrogène dans le composant eau du contenu du conteneur, une quantité connue d'eau lourde ayant été introduite dans le conteneur suffisamment longtemps avant l'extraction pour qu'il se produise une égalisation isotopique au sein des déchets, et
b) mesurer les rapports D/(H+D) et T/(H+D) dans l'échantillon, dans lequel la mesure du tritium dans le lot est représentée par la formule :

$$\text{Tritium total dans le lot de déchets} = (D \text{ dans l'échantillon}) \times \left\{ \frac{T/(H+D)}{D/(H+D)} \right\}$$

**3.** Procédé selon la revendication 2 ou 3, dans lequel l'eau lourde est introduite dans ledit conteneur à l'aide dudit échantillonneur hygroscopique.

**4.** Procédé selon l'une quelconque des revendications 1 à 3, dans lequel ledit échantillonneur hygroscopique comprend l'une quelconque des caractéristiques suivantes :

a) il absorbe un pourcentage significatif d'eau à température ambiante, dans un intervalle d'humidité relative allant d'environ 10 à 90% ;
b) il reste sensiblement solide dans les conditions définies en a) ;
c) il permet un échange de l'eau absorbée avec l'humidité dans le reste du lot de déchets, et
d) il libère l'eau par dissolution ou chauffage modéré, ou par échange isotopique en une période de temps adéquate.

**5.** Procédé selon l'une quelconque des revendications 1 à 4, dans lequel ledit échantillonneur hygroscopique est prévu dans un récipient sensiblement poreux ou perforé.

**6.** Procédé selon la revendication 5, dans lequel ledit récipient poreux est un sachet en papier ou en tissu.

**7.** Procédé selon l'une quelconque des revendications précédentes, dans lequel ledit échantillonneur hygroscopique est prévu dans un support perforé approprié pour s'adapter dans un orifice d'entrée dudit conteneur de déchets.

**8.** Procédé selon l'une quelconque des revendications précédentes, dans lequel ledit échantillonneur hygroscopique peut être introduit dans ou ôté dudit conteneur sans rompre le scellement dudit conteneur de déchets.

**9.** Procédé selon l'une quelconque des revendications précédentes, dans lequel ledit échantillonneur hygroscopique comprend l'un quelconque parmi un sel hygroscopique, un oxyde hygroscopique, du gel de silice ou une zéolite.

**10.** Procédé selon l'une quelconque des revendications précédentes, dans lequel ledit sel hygroscopique est le chlorure de baryum.

**11.** Procédé selon l'une quelconque des revendications précédentes, dans lequel ledit conteneur de déchets scellable comprend un sac ou un tambour.